# EUROPÄISCHE PATENTANMELDUNG

(11) **EP 2 179 698 A1**
(43) Veröffentlichungstag der Anmeldung: **28.04.2010**
(21) Anmeldenummer: 09173590.2
(22) Anmeldetag: 21.10.2009
(51) Int. Cl.: A61B 17/34

(54) **Punktierungsinstrument**

(30) Priorität: 27.10.2008 DE 102008043207
(71) Anmelder: Ovesco Endoscopy AG, 72074 Tübingen (DE)
(72) Erfinder: Ho, Chi-Nghia, Dipl-Ing. (FH), 70180, Stuttgart (DE)
(74) Vertreter: Winter, Brandl, Fürniss, Hübner Röss, Kaiser, Polte Partnerschaft Patent- und Rechtsanwaltskanzlei

(57) **Zusammenfassung**

Die Erfindung betrifft ein Punktierungsinstrument mit einer Führungsröhre (2), an deren distalem Ende eine Spreizankereinrichtung (4) angeordnet ist und einer Schutzhülse (1), die zumindest den distalen Endabschnitt der Führungsröhre insbesondere im Bereich der Spreizankereinrichtung axial verschiebbar umgibt, wobei innerhalb oder längs der Führungsröhre eine Hohlnadel oder ein Gastroskop (3) mit einem zu einer Schneide (3A) geformten distalen Ende axial beweglich gelagert ist.

## Beschreibung

Die vorliegende Erfindung betrifft ein vorzugsweise gastroskopisch einführbares Punktierungsinstrument für die Schaffung eines Durchgangskanals vorzugsweise durch die Magenwand.

Aus dem Stand der Technik bspw. gemäß der US 2007/0043380 A1 ist ein Punktierungsinstrument dieser Gattung bekannt. Dieses Instrument besteht unter anderem aus einer flexiblen Führungsröhre, in der ein Gastroskop axial beweglich gelagert ist. Das Gastroskop hat an dessen distalem Ende eine Schnittkante, mittels der Gewebematerial durchtrennbar ist. Des Weiteren sind in der Führungsröhre in isolierender Weise Kanülen ausgebildet, in der jeweils Ankerdrähte gleitend gelagert sind. Diese können am distalen Endabschnitt der Führungsröhre austreten um sich ankerförmig zu verwinden. Hierfür bestehen die Ankerdrähte aus einem Material mit sogenanntem Memory-Effekt oder einem federelastischen Material, welches in entspanntem Zustand eine Hakenform annimmt.

Um mit diesem bekannten Punktierungsinstrument eine Durchgangsbohrung durch die Magenwand zu schaffen, wird die Führungsröhre gastroskopisch in den Magen eingeführt und in einem vordefinierten Bereich an der Innenseite der Magenwand platziert. Anschließend werden die Ankerdrähte axial in Richtung distalem Ende der Führungsröhre verschoben und treten dabei aus dem distalen Ende der Führungsröhre heraus. Hierbei dringen die Ankerdrähte in die Magenwand ein und verhaken sich dort. Damit ist die Führungsröhre an der Mageninnenwand fixiert. Abschließend wird das Gastroskop durch die Führungsröhre nach vorne gedrückt, wobei die distale Schnittkante des Gastroskops die Magenwand aufschneidet. Schließlich kann die Öffnung geschoben werden, um den Magendurchbruch abzudichten.

Die vorstehende Konstruktion benötigt demzufolge die Schaffung von voneinander separierten Kanülen zur Aufnahme der Ankerdrähte, wodurch die Herstellung der Führungsröhre insgesamt verkompliziert und verteuert wird. Darüber hinaus müssen die Ankerdrähte möglichst reibungslos in den Kanülen geführt sein, was hohe Anforderungen an die Oberflächenqualität sowohl der Drähte wie auch der Kanülen stellt.

Angesichts dieser Sachlage ist es die Aufgabe der vorliegenden Erfindung, ein gattungsgemäßes Punktierungsinstrument bereit zu stellen, welches einfacher und damit kostengünstiger herstellbar ist und darüber hinaus eine zuverlässige Funktion gewährleistet.

Diese Aufgabe wird durch ein Punktierungsinstrument mit den Merkmalen des Patentanspruchs 1 gelöst. Weitere vorteilhafte Ausgestaltungen der Erfindung sind Gegenstand der übrigen Unteransprüche.

Der Kern der Erfindung besteht demzufolge darin, die Führungsröhre selbst mit einer Ankereinrichtung, vorzugsweise einstückig oder daran fest fixiert an deren distalem Ende zu versehen und diese Ankereinrichtung mit einer Schutzhülle zu ummanteln. Wird demzufolge die Schutzhülle entfernt, beispielsweise durch Zurückziehen der Schutzhülle oder durch Vorwärtsschieben der Führungsröhre entfaltet sich die Ankereinrichtung vorzugsweise selbsttätig und verankert sich im Gewebe des Patienten.

Dieses Konstruktionsprinzip kann gegenüber dem Stand der Technik wesentlich gröber praktisch umgesetzt werden, d. h. die einzelnen Funktionselemente haben einen weniger fragilen Aufbau, und sind daher kostengünstiger herstellbar. Darüber hinaus sind gegenüber den genannten Stand der Technik größere Tolleranzen bei der Maßhaltigkeit der einzelnen Elemente erlaubt, sodass die insgesamt die Funktionsfähigkeit erhöht wird.

Die Erfindung wird nachstehen anhand eines bevorzugten Ausführungsbeispiels unter Bezugnahme auf die begleitenden Zeichnungen näher erläutert.
Fig. 1 zeigt den Längsschnitt eines distalen Endbereichs des erfindungsgemäßen Punktierungsinstruments in zurückgezogener Position, d. h. in einem Zustand geeignet für ein gastroskopisches Einführen in den Innenraum eines Magens,

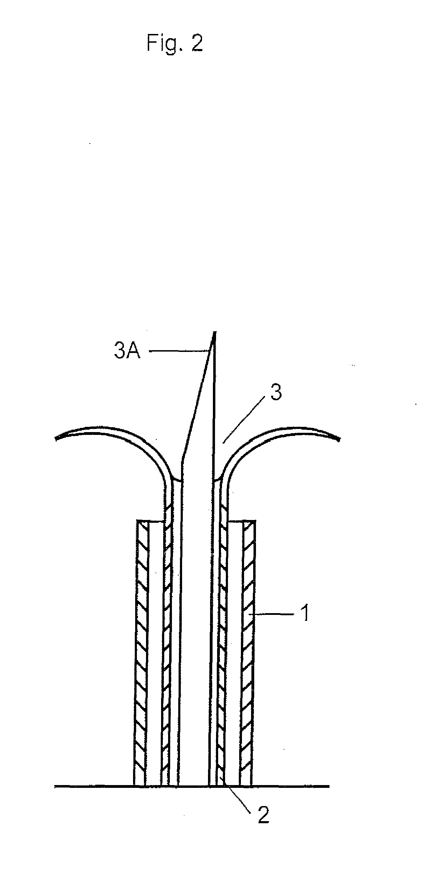

Fig. 2 zeigt das erfindungsgemäße Punktierungsinstrument in ausgefahrenem Zustand, d. h. in einem Zustand, in welchem die Herstellung eines Magenwanddurchgangs ermöglicht wird und

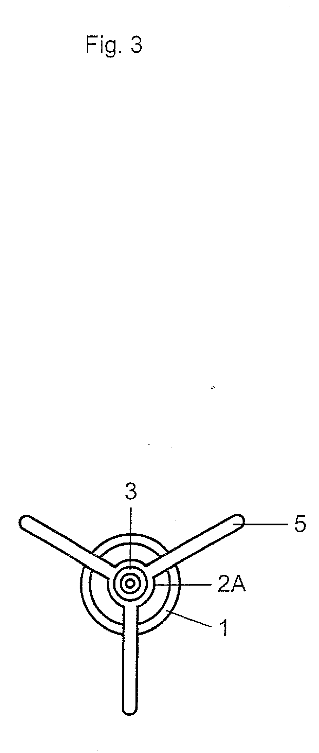

Fig. 3 zeigt die Draufsicht auf das erfindungsgemäße Punktierungsinstrument in ausgefahrenem Zustand.

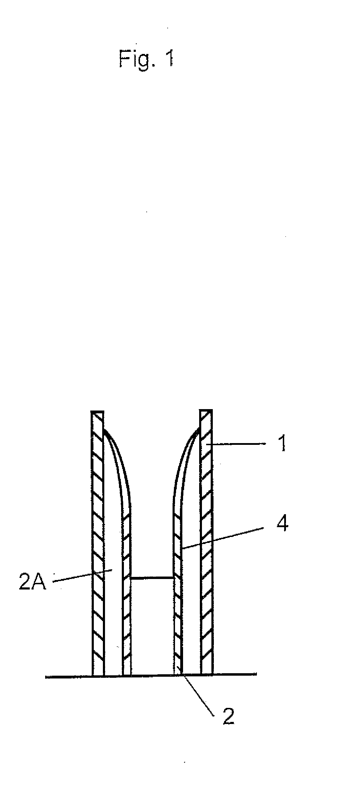

Gemäß der Fig.1 besteht das erfindungsgemäße Punktierungsinstrument nach einem bevorzugten Ausführungsbeispiel der Erfindung aus einer Führungsröhre 2, an deren distalem Ende eine Art Spreizanker 4 vorzugsweise einstückig ausgebildet ist und einer Anker- oder Schutzhülse 1, die zumindest den distalen Endabschnitt der Führungsröhre 2 insbesondere im Bereich des Spreizankers 4 axial verschiebbar umgibt. Innerhalb der Führungsröhre 2 ist eine Hohlnadel oder ein Gastroskop mit einem zu einer Schneide geformten distalen Ende axial oder achsparallel beweglich gelagert. Alternativ hierzu ist es aber auch möglich, für den Fall dass der Spreizanker besonders klein dimensioniert ist, die Hohlnadel oder das Gastroskop außerhalb der Führungsröhre parallel zu dieser zu führen, wobei in diesem Fall die Hohlnadel bzw. das Gastroskop vorzugsweise von der Schutzhülse direkt umgeben wird.

Im Einzelnen besteht der Spreizanker 4 aus vorzugsweise drei, in gleichen Umfangsabständen angeordneten Nadeln oder Krallen 5, welche federelastisch im entspannten Zustand radial nach außen gebogen sind. Die Nadeln oder Laschen 5 gehen dabei an deren Wurzeln einstückig in ein hülsenförmiges Grundelement 2A des Spreizankers 2 über, wodurch ein hülsenförmiger Hohlraum geschaffen wird. Das Hülsenelement 2A ist ferner vorzugsweise einstückig mit dem distalen Ende der Führungsröhre 2 koaxial, vorzugsweise stoßförmig verbunden, um hierdurch einen durchgehenden Führungskanal auszubilden.

Innerhalb des Führungskanals befindet sich axial oder achsparallel (d. h. dezentral) relativ zur Führungsröhre 2 bewegbar eine Hohlnadel oder ein Gastroskop 3, welches sich längs der Führungsröhre 2 erstreckt. Die Hohlnadel bzw. das Gastroskop 3 sowie die Führungsröhre 2 sind dabei flexibel dargestellt, dass sie bspw. durch die Speiseröhre eines Patienten in den Magen vorgeschoben werden können. Die Hohlnadel bzw. das Gastroskop 3 hat an dessen distalem Ende Gewebematerial, bspw. einer Magenwand.

Die zumindest den Spreizanker 4 umgebende Hülse 1 ist ebenfalls axial beweglich bezüglich der Führungsröhre 2 gelagert und erstreckt sich längs der Führungsröhre 2 sowie der darin liegenden Hohlnadel bzw. dem darin befindlichen Gastroskop 3. Vorzugsweise erstreckt sich die äußere Hülse 1 über die gesamte Länge der Führungsröhre 2, sodass sie am proximalen Ende des Punktierungsinstruments betätigbar ist. Alternativ ist es jedoch auch möglich, dass sich die Führungshülse 1 lediglich im distalen Endbereich axial längs des Spreizankers 4 erstreckt und von einem achsparallel zur Führungsröhre 2 verlaufenden Zug-Druck-Element, bspw. einem Bautenzug betätigbar ist.

Die Funktion des erfindungsgemäßen Punktierungsinstruments lässt sich dabei wie folgt umschreiben.

Das Punktierungsinstrument lässt sich aufgrund der Flexibilität der Führungsröhre 2, der darin gelagerten Hohlnadel bzw. Gastroskop 3 sowie der die Führungsröhre umgebenen Hülse 1 durch die Speiseröhre in den Magen eines Patienten einführen. Sobald das distale Ende des Punktierungsinstruments die Magenwand an einer vorbestimmten Stelle erreicht hat bzw. diese berührt, wird die Führungsröhre 2 weiter nach vorne geschoben, wobei jedoch die die Führungsröhre 2 zumindest an deren distalem Endabschnitt umgebenden Hülse 1 zurück gehalten wird. Demzufolge werden die Nadeln bzw. spitz laufenden Laschen 5 des Spreizankers 4 am distalen Ende der Führungsröhre 2 axial freigelegt, d. h. sie ragen fortlaufend über das distale Ende der Schutzhülse 1 vor, wobei mit weiterem Fortschreiten der Vorwärtsbewegung der Führungsröhre 2 die Nadeln oder Laschen 5 sich aufgrund der Federvorspannung oder eines "memory-Effekts" im Material radial nach außen aufspreizen. Bei dieser Bewegung dringen die Nadeln oder Laschen 5 in das Gewebe der Magenwand ein und verankern sich dort in Folge ihrer kontinuierlich radial aufspreizenden Verformung. Im vollständig ausgefahrenen Zustand erhalten demzufolge die Nadeln bzw. Laschen 5 eine kreisbogenartige Form, wodurch eine Zug- bzw. Druckkraft in Axialrichtung der Führungsröhre 2 in die Magenwand einleitbar ist. Das heißt, dass beispielsweise die Magenwand über die Führungsröhre 2 nach innen gezogen werden kann, wodurch sich an der Außenseite der Magenwand im Aufspreizbereich der Nadeln oder Laschen 5 des Spreizankers ein Hohlraum ausbildet.

Abschließend wird die hohlförmige Nadel bzw. das Gastroskop 3 innerhalb der Führungsröhre 2 nach vorne geschoben, wobei die Schneidkante am distalen Ende der Hohlnadel bzw. des Gastroskops in das Magenwandgewebe einschneidet, um hierdurch eine Durchgangsöffnung von der Magenwandinnenseite bis zur Magenwandaußenseite zu schaffen. Aufgrund der besonderen Formgebung des Spreizankers insbesondere der federelastischen Nadeln oder Laschen, welche sich beim Vorschieben des Spreizankers selbsttätig radial aufspannen ist es möglich, wie vorstehend bereits beschrieben, die Magenwand zurück zu ziehen, um hierdurch an der Magenwandaußenseite einen kreisförmigen Hohlraum zu bilden. Hierdurch kann verhindert werden, dass beim Durchstoßen der Magenwand mittels der hohlförmigen Nadel bzw. des die Schneidkante aufweisenden Gastroskops eine Verletzung umliegenden Gewebes erfolgt.

Die vorstehende Beschreibung zeigt, dass durch die Ausrüstung der Führungsröhre mit dem Spreizanker die Ausbildung zusätzlicher Führungskanülen innerhalb der Führungsröhre entfällt. Auf diese Weise lässt sich die Führungshülse auf eine einfache Geometrie und damit eine simple Herstellungsweise reduzieren. Des Weiteren ist durch die Anordnung einer zumindest den Spreizanker umgebenden Hülse oder Kappe gewährleistet, dass eine Verletzung von Gewebe in zurückgezogenem Zustand des Spreizankers sicher vermieden wird. Darüber hinaus ist die Herstellung einer derartigen Hülse oder Schutzkappe /-hülse einfach und damit kostengünstig. Schließlich hat sich gezeigt, dass aufgrund der gegenüber dem genannten Stand der Technik weniger filigranen Bauweise sämtlicher Bauteile die Funktionssicherheit steigert und damit die Funktionalität des Instruments verbessert werden kann.

An dieser Stelle seien auch einige Abwandlungen des vorstehend beschriebenen ersten Ausführungsbeispiels genannt:

Der Spreizanker wird im vorliegenden Ausführungsbeispiel einstückig mit der Führungshülse ausgebildet. Alternativ hierzu ist es jedoch auch möglich, den Spreizanker als ein zur Führungsröhre separat gefertigtes Bauteil vorzusehen, welches anschließend am distalen Ende der Führungsröhre angeschraubt, angeschweißt, angelötet oder angeklebt wird. Die Fixierstelle kann hierbei stoßweise oder in überlappender Weise erfolgen.

Die Nadeln bzw. Spitzen oder scharfkantigen Laschen den Spreizankers sind im bevorzugten Ausführungsbeispiel federelastisch. Alternativ hierzu kann jedoch auch ein Material mit sogenanntem Memory-Effekt verwendet werden.

## Patentansprüche

1. Punktierungsinstrument mit einer Führungsröhre, an deren distalem Ende eine Spreizankereinrichtung angeordnet ist und einer Schutzhülse, die zumindest den distalen Endabschnitt der Führungsröhre insbesondere im Bereich der Spreizankereinrichtung axial verschiebbar umgibt, wobei innerhalb oder längs der Führungsröhre eine Hohlnadel oder ein Gastroskop mit einem zu einer Schneide geformten distalen Ende axial beweglich gelagert ist.

2. Punktierungsinstrument nach Anspruch 1, **dadurch gekennzeichnet, dass** die Spreizankereinrichtung aus einem becher- oder hülsenartigen Grundelement besteht, an dem axial sich erstreckende Nadeln oder spitze Laschen ausgebildet sind, die federelastisch oder nach dem Prinzip eines memory-Effekts in eine radial nach Außen gekrümmte Form vorgeformt sind.

3. Punktierungsinstrument nach Anspruch 2, **dadurch gekennzeichnet, dass** die Spreizankereinrichtung einstückig mit der Führungsröhre ausgebildet ist.

4. Punktierungsinstrument nach einer der vorstehenden Ansprüche, **dadurch gekennzeichnet, dass** die Führungsröhre aus einem biegeflexiblen Material besteht.

5. Punktierungsinstrument nach einem der vorstehenden Ansprüche, **dadurch gekennzeichnet, dass** die Hohlnadel das distale Endstück eines elastischen Schlauchelements bildet, welches sich innerhalb der Führungsröhre und der Spreizankereinrichtung axialverschieblich erstreckt.

6. Punktierungsinstrument nach einem der vorstehenden Ansprüche, **dadurch gekennzeichnet, dass** die Schutzhülse aus einem biegeflexiblen Material besteht und sich längs der Führungsröhre erstreckt.

7. Punktierungsinstrument nach einem der Ansprüche 1 bis 5, **dadurch gekennzeichnet, dass** am proximalen Ende der Schutzhülse ein Zug-/Druckelement, vorzugsweise ein Betätigungsdraht angebracht ist, der sich längs der Führungsröhre erstreckt.

8. Punktierungsinstrument nach Anspruch 1, **dadurch gekennzeichnet, dass** die Hohlnadel oder das Gastroskop längs der Führungsröhre außerhalb von dieser jedoch innerhalb der Schutzhülse gelagert ist.

## Geänderte Patentansprüche

### Geänderte Patentansprüche gemäss Regel 137(2) EPÜ.

**1.** Punktierungsinstrument mit einer Führungsröhre (2), an deren distalem Ende eine Spreizankereinrichtung (4) angeordnet ist und einer Schutzhülse (1), die zumindest den distalen Endabschnitt der Führungsröhre (2) insbesondere im Bereich der Spreizankereinrichtung (4) axial verschiebbar umgibt, **dadurch gekennzeichnet, dass** innerhalb oder längs der Führungsröhre (2) eine Hohlnadel oder ein Gastroskop (3) mit einem zu einer Schneide (3A) geformten distalen Ende axial beweglich gelagert ist, wobei die Spreizankereinrichtung (4) aus einem becher- oder hülsenartigen Grundelement besteht, an dem axial sich erstreckende Nadeln oder spitze Laschen ausgebildet sind, die federelastisch oder nach dem Prinzip eines memory-Effekts in eine radial nach Außen gekrümmte Form vorgeformt sind, wobei das Grundelement mit dem distalen Ende der Führungsröhre (2) verbunden ist und wobei die Führungsröhre (2) sowie die Hohlnadel oder das Gastroskop (3) aus einem biegeflexiblen Material bestehen.

**2.** Punktierungsinstrument nach einem der vorstehenden Ansprüche, **dadurch gekennzeichnet, dass** die Hohlnadel das distale Endstück eines elastischen Schlauchelements bildet, welches sich innerhalb der Führungsröhre und der Spreizankereinrichtung axialverschieblich erstreckt.

**3.** Punktierungsinstrument nach einem der vorstehenden Ansprüche, **dadurch gekennzeichnet, dass** die Schutzhülse (1) aus einem biegeflexiblen Material besteht und sich längs der Führungsröhre (2) erstreckt.

**4.** Punktierungsinstrument nach einem der Ansprüche 1 bis 2, **dadurch gekennzeichnet, dass** am proximalen Ende der Schutzhülse (1) ein Zug-/Druckelement, vorzugsweise ein Betätigungsdraht angebracht ist, der sich längs der Führungsröhre (2) erstreckt.

**5.** Punktierungsinstrument nach Anspruch 1, **dadurch gekennzeichnet, dass** die Hohlnadel oder das Gastroskop (3) längs der Führungsröhre (2) außerhalb von dieser jedoch innerhalb der Schutzhülse (1) gelagert ist.
